# FASCICULE DE BREVET EUROPEEN

(11) **EP 0 510 168 B1**
(45) Date de publication et mention de la délivrance du brevet: **08.01.1997**
(21) Numéro de dépôt: 92900281.4
(22) Date de dépôt: 15.11.1991
(51) Int. Cl.: C07D 233/74

(54) **PROCEDE DE DEDOUBLEMENT D'HYDANTOINES CHIRALES**
VERFAHREN ZUR RACEMATGELTUNG VON CHIRALEN HYDANTOINEN
CHIRAL HYDANTOIN RESOLUTION

(30) Priorité: 16.11.1990 FR 9014487
(43) Date de publication de la demande: 28.10.1992
(73) Titulaire: JOUVEINAL S.A., F-75755 Paris Cedex 15 (FR)
(72) Inventeur: DEPERNET, Dominique, F-49320 Brissac-Quince (FR); BOUAZIZ, Roger, F-75116 Paris (FR); COQUEREL, Gérard, F-76960 Notre-Dame-de-Bondeville (FR); PETIT, Marie-Noelle, F-76130 Mont-Saint-Aignan (FR)
(74) Mandataire: Bourgognon, Jean-Marie
(86) Numéro de dépôt international: FR9100906
(87) Numéro de publication internationale: WO9208702

(56) Documents cités:
- CHEMICAL ABSTRACTS, vol. 106, no. 18, 4 Mai 1987, Columbus, Ohio, US; abstract no. 144071D, MAGUIRE JAMES H.: 'SOME STRUCTURAL REQUIREMENTS FOR RESOLUTION OF HYDANTOIN ENANTIOMERS WITH A BETA-CYCLODEXTRIN LIQUID CHROMATOGRAPHY COLUMN.' page 376 ;colonne 2; & J. CHROMATOGR. no. 387, 1987, AMSTERDAM pages 453-458;

## Description

La présente invention a pour objet un procédé de dédoublement d'hydantoïnes chirales tel que défini à la revendication 1.

On sait que le dédoublement des molécules chirales fait classiquement appel à la formation de sels diastéréoisomères dont les différences de solubilité permettent la séparation de l'un d'entre eux, qui est ensuite décomposé pour conduire à l'énantiomère choisi.

On sait également que ces méthodes font appel à des agents de résolution optiquement actifs souvent toxiques ou instables, qui sont proposés dans le commerce à l'échelle industrielle sous une forme de pureté très relative et/ou coûteuse et que, en outre, il n'est souvent proposé qu'une seule forme énantiomère de l'agent de résolution.

On connaît par ailleurs l'intérêt des énantiomères d'hydantoïnes chirales, et notamment celui présenté par les énantiomères des hydantoïnes de formule (I).

Ainsi l'hydantoïne racémique (I) dans laquelle R₁ est un radical éthyle et R₂ un radical phényle a été utilisée pour ses propriétés anticonvulsivantes dans le traitement de l'épilepsie sous le nom de "phénytoïne" ou encore de "Nirvanol" (DCI). Son analogue N-méthylé en position 3 du cycle hydantïne possède les mêmes propriétés et a été utilisé sous le nom de "méphénytoïne". Or, entre autres auteurs, Kuepfer et coll. (J. Pharmacol. Exp. Ther. 221 (3), 590-7, 1982 et 230 (1), 28-33, 1984) ont constaté chez l'homme, pour les énantiomères de ces deux composés, des différences de biodisponibilité et de métabolisme qui peuvent être exploitées pour l'amélioration du traitement des crises convulsives.

Par ailleurs, les hydantoïnes chirales (I), qui sont facilement préparées à partir des dérivés carbonylés R₁-CO-R₂ correspondants par la réaction de Bucherer-Berg, sont des intermédiaires de choix pour la préparation d'énantiomères d'amino acides de formule générale (II) : 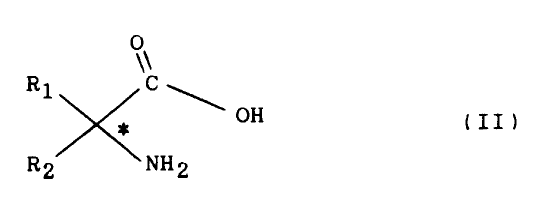 dans laquelle R₁ et R₂ ont les significations précédemment indiquées pour les hydantoïnes (I).

Certains de ces énantiomères montrent d'intéressantes propriétés anti-hypertensives, comme notamment la "Méthyldopa" (DCI) qui est l'énantiomère lévogyre de l'amino acide dans lequel R₁ est un radical méthyle et R₂ un radical 3,4-dihydroxybenzyle. D'autres amino acides optiquement actifs sont des intermédiaires de choix pour la préparation de composés thérapeutiquement actifs.

Ainsi, les énantiomères et le racémique de l'amino acide dans lequel R₁ est un radical méthyle et R₂ un radical 3,4-dichlorobenzyle conduisent, après réduction par des hydrures métalliques ou organo métalliques, à des amino-alcools dont certains, N-substitués, décrits dans le document FR-A-86 01 295, possèdent des propriétés-analgésiques et sont actifs sur le système nerveux central.

Egalement, les amino acides optiquement actifs dans lesquels R₁ est un radical éthyle et R₂ soit un radical phényle soit un radical thiényle permettent, après leur réduction en amino-alcools, d'obtenir des amino-esters ou des amino-éthers actifs sur le tractus gastro intestinal, comme certains des composés décrits dans les documents FR-A-2 643 369 et EP-A-0 297 782.

Pour réaliser le dédoublement des hydantoïnes chirales (I) différentes méthodes ont été proposées. Ainsi, on a proposé des méthodes de cristallisation préférentielle qui nécessitent un ensemencement cristallin avec une forme énantiomère préalablement obtenue et qui sont, généralement, de rendement médiocre. On a également proposé des techniques chromatographiques sur support chiral peu adaptées à l'industrie et particulièrement coûteuses. enfin, on a proposé diverses méthodes faisant appel à la formation de sels diastéréoisomères avec des énantiomères d'amines diverses qui sont le plus souvent coûteuses tout en étant de stabilité et de pureté industrielle douteuses.

On connaît au document ES-A-8701180 le dédoublement d'une hydantoïne de formule 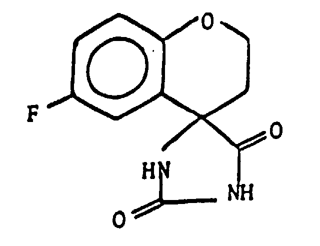 par cristallisation fractionnée de son sel dans un alcool avec la L-α-méthylbenzylamine.

On connaît également d'après le document Journal of Chromatography, 387, 1987, pages 453 à 458, le dédoublement d'hydantoïne par utilisation de la β-cyclodextrine.

Aucune de ces méthodes n'est appropriée à une application industrielle, fiable et économique, du dédoublement des hydantoïnes chirales de formule (I).

La présente invention permet de remédier à ces inconvénients en proposant un procédé de dédoublement qui fait appel à un agent de résolution optiquement actif d'usage courant, l'α-méthylbenzylamine, sous la forme de ses isomères (R)(+) et (S) (-), tous deux commercialement disponibles, et dont les propriétés physico chimiques (solubilité, point d'ébullition) en permettent une récupération et un recyclage industriel aisé.

Le procédé selon l'invention est caractérisé par la partie caractérisante de la revendication 1.

On obtient ainsi l'énantiomère de l'hydantoïne (I) dans des conditions de rendement et de pureté optique satisfaisantes.

Un premier mode de réalisation du procédé selon l'invention consiste à dissoudre l'hydantoïne chirale (I) à dédoubler dans une solution alcaline puis à ajouter l'énantiomère de l'α-méthylbenzylamine à raison d'au moins un équivalent molaire pour une mole d'hydantoïne à dédoubler, en vue d'obtenir la cristallisation d'un sel diastéréoisomère,puis à traiter ce dernier selon le mode opératoire précédemment décrit pour obtenir un des énantiomères de l'hydantoïne (I).

Dans ce premier mode de réalisation du procédé selon l'invention, la solution alcaline peut être :
- soit une solution aqueuse contenant de l'ammoniaque, un hydroxyde minéral ou une base organique hydrosoluble ;
- soit une solution aqueuse d'un hydroxyde minéral associée à un co-solvant de point d'ébullition inférieur à 100° C et qui peut être un alcool hydrosoluble comprenant de 1 à 3 atomes de carbone, ou, préférentiellement, l'acétone.

Dans le cas où la solution alcaline est une solution aqueuse ammoniacale, il convient de mettre en oeuvre au moins trois équivalents molaires d'ammoniaque pour obtenir des résultats convenables tant au point de vue de la pureté optique que du rendement pondéral.

Dans le cas où la solution alcaline est une solution aqueuse d'un hydroxyde minéral, celui-ci est préférentiellement un hydroxyde de métal alcalin et plus particulièrement l'hydroxyde de sodium. Dans ce cas, pour un équivalent molaire d'hydantoïne chirale (I) le dédoublement est réalisé en présence d'environ 0,5 équivalent molaire d'hydroxyde de sodium et en ajoutant de un à trois équivalents molaires de l'énantiomère d'α-méthylbenzylamine.

Toutefois le procédé préférentiellement mis en oeuvre est celui qui fait intervenir un hydroxyde minéral et un énantiomère d'α-méthylbenzylamine en milieu aqueux alcalin auquel on associe comme co-solvant préféré l'acétone, à raison de 0,2 à 10 volumes pour un volume de solution aqueuse, le mélange d'un volume d'eau avec 0,5 à 2 volumes d'acétone conduisant généralement à des résultats satisfaisants.

Les exemples qui suivent sont fournis à titre d'illustration non limitative des applications du procédé objet de l'invention.

### EXEMPLE 2

### Dédoublement en solution aqueuse ammoniacale de la (+/-)5-phényl-5-éthyl-hydantoïne (désignée par l'abréviation dans tous ce qui suit)

Dans un ballon de 250 ml à fond plat, on introduit environ 2,04 g de (+/-) PEHYD (0,01 mole) en solution ammoniacale de quantité tel qu'indiquée au tableau récapitulatif qui suit. On réalise différents essais de dédoublement avec 1,21 g (0,01 mole) de (R) (+) -α-méthylbenzylamine.

L'énantiomère obtenu est l'isomère dextrogyre (+) PEHYD.

Le sel diastéréoisomère qui précipite est filtré, lavé à l'eau puis traité par une solution de HC1 N excès afin d'obtenir l'énantiomère de PEHYD.

La précipitation est complétée sous agitation à 10° pendant deux heures. L'insoluble est filtré, lavé à l'eau et séché jusqu'à poids constant par dessication sous vide à 50°C.

La détermination du pouvoir rotatoire du produit en solution dans l'éthanol (c = 2) montre que le produit obtenu est l'énantiomère dextrogyre de la PEHYD.

La valeur du pouvoir rotatoire trouvée dans ces conditions est comparée à celle déterminée par Sobotka et coll. (J. Am. Chem. Soc., 1932, 54, 4697-702) : [α]_{D} = +123° (c environ 2% dans EtOH) pour déterminer en % la pureté optique du produit obtenu.

Les essais et les résultats obtenus sont reportés dans le tableau qui suit, dans lequel le rendement exprimé en % correspond au produit du rendement massique du dédoublement en % par la pureté optique en % de l'énantiomère obtenu.

Les conditions des essais et les résultats obtenus sont récapitulés dans le tableau qui suit.

| Exemple | V-eau (ml) | Moles NH₃/Moles(+/-)PEHYD | (+) PEHYD obtenu(g) | Pureté optique(%) | Rendement (%) |
|---|---|---|---|---|---|
| 2-a | 70 | 3 | 0,81 | 73 | 58 |
| 2-b | 80 | 3 | 0,89 | 63 | 55 |
| 2-c | 90 | 3 | 0,85 | 71 | 60 |
| 2-d | 100 | 3 | 0,58 | 99 | 56 |
| 2-e | 70 | 6 | 0,59 | 97 | 56 |

Ces essais sont probants de l'efficacité du dédoublement réalisé en solution ammoniacale, notamment les essais 2-d et 2-e qui conduisent à l'obtention d'un produit de pureté optique élevée.

### EXEMPLE 3

### Dédoublement de la (+/-) PEHYD en solution aqueuse d'hydroxyde de sodium.

En pratiquant selon le mode opératoire de l'exemple 2 à partir de 0,01 mole de (+/-) PEHYD et 0,01 mole de (R)(+)α-méthylbenzylamine, en faisant varier le rapport moles NaOH/moles (+/-PEHYD, on réalise les essais des exemples 3-a à 3-i dont les conditions et les résultats sont indiqués dans le tableau qui suit, l'énantiomère isolé étant la (+)PEHYD.

| Exemple | V.eau (ml) | Moles NaOH/Moles(+/-)PEHYD | (+) PEHYD obtenu(g) | Pureté optique(%) | Rendement (%) |
|---|---|---|---|---|---|
| 3-a | 110 | 0,125 | 1,21 | 23 | 27 |
| 3-b | 110 | 0,166 | 1,15 | 25 | 28 |
| 3-c | 110 | 0,250 | 1,06 | 40 | 42 |
| 3-d | 110 | 0,33 | 0,96 | 58 | 54 |
| 3-e | 110 | 0,40 | 0,85 | 71 | 61 |
| 3-f | 110 | 0,45 | 0,74 | 91 | 65 |
| 3-g | 110 | 0,50 | 0,63 | 96 | 60 |
| 3-h | 110 | 0,60 | 0,45 | 97 | 43 |
| 3-i | 110 | 0,75 | 0,38 | 98 | 37 |

Les essais des exemples 3-g à 3-i dans lesquels l'hydroxyde de sodium est utilisé à raison de 0,50 à 0,75 équivalents par rapport à la (+/-) PEHYD, permettent d'obtenir l'énantiomère (+) avec une pureté optique satisfaisante, toutefois au détriment du rendement avec les quantités plus importantes d'hydroxyde de sodium (exemple 3-i).

### EXEMPLE 4

Selon le mode opératoire de l'exemple 3-g précédent les exemples 4-a et 4-b sont réalisés en modifiant la quantité de (R)(+)-α-méthylbenzylamine engagée. Les résultats de ces essais comparés à celui de l'exemple 3.g sont indiqués dans le tableau ci-après.

| Exemple | MolesNaOH/MolesPEHYD | Moles(R/+)amine/Moles(+/-)PEHYD | (+) PEHYD obtenu (g) | Pureté optique(%) | Rdt (%) |
|---|---|---|---|---|---|
| 4-a | 0,50 | 0,5 | 0,79 | 30 | 23 |
| 3-g | 0,50 | 1,0 | 0,63 | 96 | 60 |
| 4-b | 0,50 | 2,0 | 0,67 | 99 | 66 |

Les essais des exemples 3 et 4 mettent en évidence que les meilleurs résultats sont obtenus en mettant en oeuvre, pour le dédoublement d'une mole de (+/-) PEHYD), 2 équivalents molaires d'α-méthylbenzylamine et 0,5 équivalent molaire de NaOH, encore que d'excellents résultats soient obtenus avec un équivalent molaire d'α-méthylbenzylamine et 0,45 à 0,5 équivalent molaire de NaOH.

### EXEMPLE 5

Selon le mode opératoire de l'exemple 3 on applique le procédé de dédoublement en solution aqueuse d'hydroxyde de sodium à diverses hydantoïnes chirales (I).

Chaque dédoublement est réalisé à partir de 0,01 mole d'hydantoïne chirale (I) avec la (R)(+)α-méthylbenzylamine.

Les énantiomères d'hydantoïnes obtenus après dédoublement provoquent en solution dans l'éthanol une déviation dextrogyre.

Les produits soumis au dédoublement, les conditions des essais et les résultats obtenus sont reportés dans le tableau ci-après.

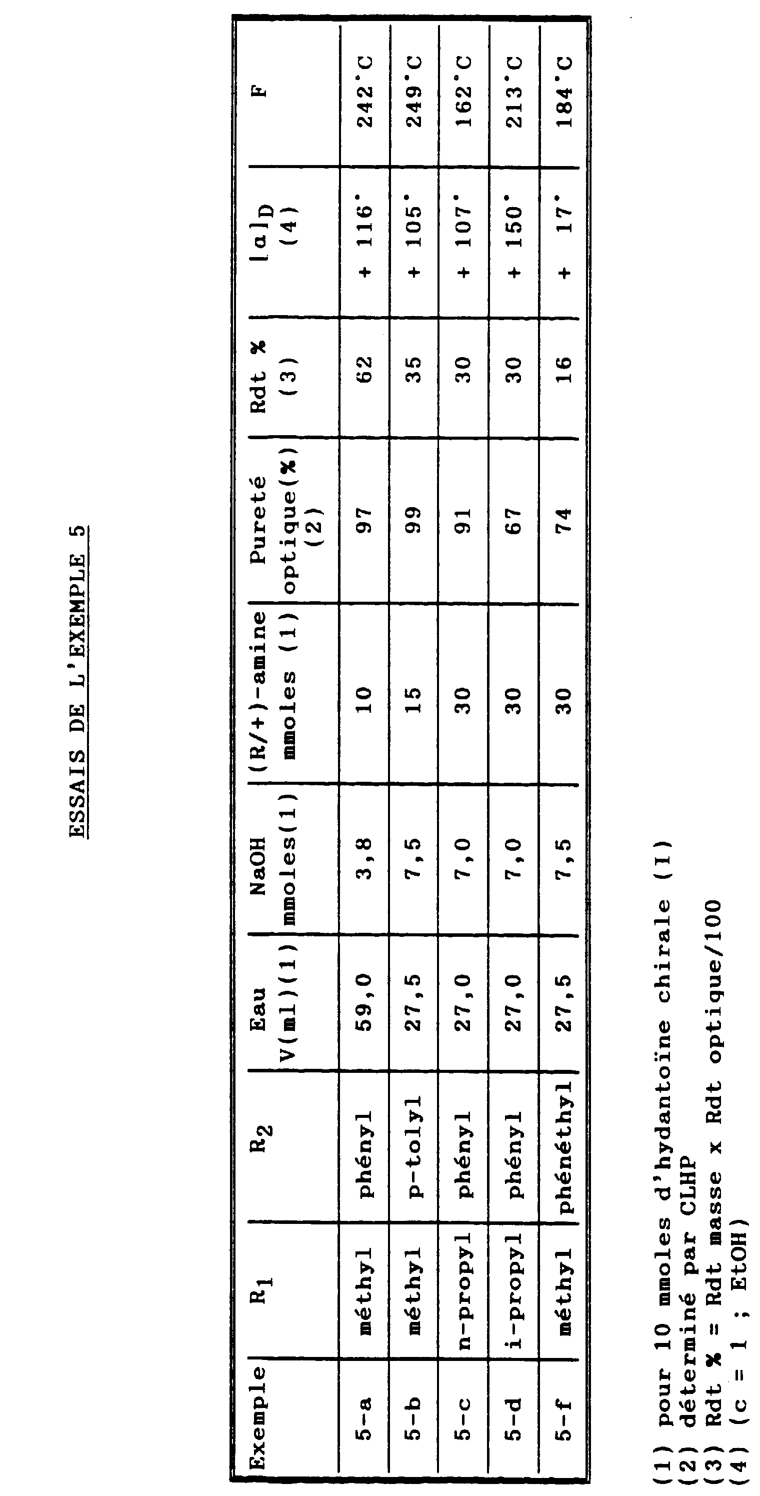

### EXEMPLE 6

### Dédoublement de la (+/-) PEHYD en solution aqueuse d'hydroxyde de sodium, avec l'acétone comme co-solvant.

Dans un réacteur équipé en position de reflux on solubilise 20,0 g (0,098 mole) de (+/-) PEHYD dans 200 ml d'un mélange acétone/eau (V/V). On ajoute 1,76 g (0,044 mole) d'hydroxyde de sodium en pastilles puis 12,2 g (0,098 mole) de (S)(-)α-méthylbenzylamine.

La suspension est agitée en chauffant progressivement pour obtenir à 65° C la dissolution complète des réactifs, puis la solution est refroidie lentement jusqu'à une température d'environ 40° C, à laquelle une cristallisation s'amorce. Après avoir maintenu une heure à cette température pour favoriser le développement cristallin, le milieu est refroidi progressivement jusqu'à 20-25° C, puis abandonné une nuit sous agitation.

L'insoluble est filtré, lavé par 100 ml d'eau et, sous agitation et sans dépasser 20° C, la suspension est acidifiée jusqu'à pH 1 par addition de 4,0 ml d'acide chlorhydrique concentré, provoquant la précipitation de l'énantiomère lévogyre de la PEHYD qui est filtré, lavé à l'eau puis séché sous vide à 50° C.

On obtient 5,4 g de (S)(-)PEHYD de point de fusion 241,4° C et de pureté optique (CLPH) égale à 100 %, le rendement de l'opération étant de 54 %.

### EXEMPLE 7

### Dédoublement de la (+/-)5-(2-thiényl)-5-éthyl hydantoïne (R₁ = éthyl R₂ = 2-thiényl) en solution aqueuse d'hydroxyde de sodium.

Dans un réacteur d'un litre, on ajoute 10,5 g (0,05 mole) de (+/-)5-(2-thiényl)-5-éthyl hydantoïne à 550 ml d'eau déminéralisée. Sous agitation, on introduit à la suspension 2,5 ml de solution 10 N d'hydroxyde de sodium (0,025 mole). Après 10 minutes, on obtient une solution à laquelle on ajoute goutte à goutte, à 25-30°, 6,1 g (0,05 mole) de (S)(-)-α-méthylbenzylamine.

En maintenant sous agitation et en refroidissant progressivement à 10° C, une cristallisation apparaît lentement.

Après une nuit sous agitation à cette température, le sel diastéréoisomère cristallin est filtré, mis en suspension sous agitation dans une solution d'acide chlorhydrique N, durant 15 minutes.

L'énantiomère obtenu est la (S)(-)5-(2-thiényl)-5-éthyl hydantoïne, qui est filtrée, lavée à l'eau et séchée sous vide à 50° C.
Poids obtenu = 1,35 g Rdt chimique = 26 %
[α]_{D} = - 135° (c=1, EtOH)
Pureté optique = 99 % (CLPH)
Rendement = 25 %

### EXEMPLE 8

### Dédoublement de la (+/-)5-(3,4-dichlorobenzy)-5-méthyl hydantoïne (R₁ = méthyl, R₂ = 3, 4-dichlorobenzyl) en solution aqueuse d'un hydroxyde minéral associée à l'acétone.

### a) Préparation de l'hydantoïne racémique

Dans un réacteur, on introduit 160,0 g (0,79 mole) de 3,4-dichlorophénylacétone dans 700 ml d'éthanol et on chauffe le mélange à 40° C pour obtenir la dissolution. On ajoute ensuite 53,0 g (0,81 mole) de cyanure de potassium, 149,0 g de sesquicarbonate d'ammonium et 700 ml d'eau.

Le mélange est chauffé sous agitation à une température comprise entre 65 et 70° C durant 15 heures. La suspension est refroidie sous agitation à 10-15° C et abandonnée 16 heures à cette température. L'insoluble est filtré, lavé à l'eau puis à l'éther di-isopropylique avant d'être séché sous vide à 50° C jusqu'à poids constant.

On obtient 155,0 g de produit de point de fusion 240° C, le rendement de l'opération étant 72 %.

### b) Dédoublement de l'hydantoïne racémique

Dans un réacteur équipé en position de reflux, on mélange 100 ml d'eau déminéralisée avec 150 ml d'acétone puis on ajoute 3,7 g (0,091 mole) d'hydroxyde de sodium en pastilles.

On ajoute ensuite successivement 50,0 g (0,183 mole) de (+/-) 5-(3,4-dichlorobenzyl)-5-méthyl hydantoïne et 22,2 g (0,183 mole) de (R)(+)α-méthylbenzylamine.

Sous agitation le mélange est porté au reflux jusqu'à dissolution des réactifs. On introduit alors 100 ml d'eau en chauffant le mélange progressivement jusqu'à 70° C, puis on refroidit lentement la solution sous agitation.

Un début de cristallisation apparaît vers 60° C et le mélange est alors maintenu 1 h 30 à 50° C pour parfaire la cristallisation, puis refroidi à 20° C environ durant 30 minutes.

Le précipité cristallin est filtré, lavé par 2 fois 100 ml d'un mélange acétone/eau 2-1 (vol/vol).

L'insoluble est, tel quel, mis en suspension dans 250 ml d'eau et le mélange est acidifié à pH 1 par addition progressive d'une solution d'acide chlorhydrique concentré.

L'insoluble est filtré, lavé à l'eau. La pureté optique du produit déterminé par CLHP sur une fraction aliquote est de 98,6 %.

Le produit humide est recristallisé au reflux dans 200 ml d'un mélange eau-acétone 1 - 1 (vol/vol).

Après refroidissement pendant deux heures à 15° C l'insoluble est filtré, lavé par un mélange acétone-eau puis séché sou vide à 50° C.

On obtient 18,79 g de l'énantiomère (-) de la 5-(3,4-dichlorobenzyl)-5-méthyl hydantoïne.
Rendement = 75 %
Pureté optique = 100 % (CLHP)
F = 276° C [α]_{D} = - 28,4° (c=1, EtOH)

Ce procédé de dédoublement réalisé avec la (S)(-)α-méthylbenzylamine conduit à l'énantiomère (+) de la 5-(3,4-dichlorobenzyl)-5-méthyl hydantoïne.
Rendement = 52 %
Pureté optique = 100 % (CLHP)
F>260° C
[α]_{D} = + 26,5° (c = 1, EtOH)

## Revendications

1. Procédé de dédoublement d'hydantoïnes chirales qui consiste à dissoudre l'hydantoïne à dédoubler dans un solvant, à ajouter à la solution obtenue un énantiomère de l'α-méthylbenzylamine en proportion définie, puis à isoler le sel diastéréoisomère obtenu et à le traiter par une solution acide pour cristalliser l'énantiomère de l'hydantoïne, caractérisé en ce que l'hydantoïne est de formule générale (I) 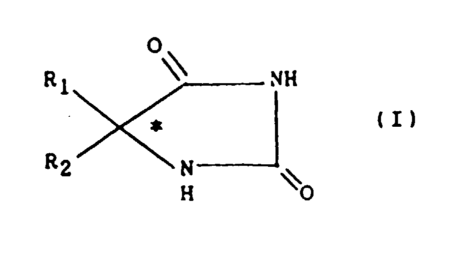 dans laquelle :
R₁ désigne un radical alkyle inférieur comprenant de 1 à 5 atomes de carbone en chaîne linéaire ou ramifiée,
R₂ désigne un radical phényle éventuellement mono, di ou trisubstitué par des radicaux alkyle ou alkoxy inférieurs comprenant de 1 à 5 atomes de carbone ou des atomes d'halogène, identiques ou différents, ou un radical hétéroaryle renfermant un cycle de 5 à 7 chaînons dans lequel l'unique hétéroatome est l'azote, l'oxygène ou le soufre, ou encore un radical aralkyle dont la partie alkyle comprend un ou deux atomes de carbone et le cycle aryle est un groupe phényle éventuellement mono, di ou trisubstitué par des radicaux qui sont alkyle ou alkoxy inférieurs ou des atomes d'halogène, identiques ou différents et le solvant est une solution alcaline.

2. Procédé selon la revendication 1, caractérisé en ce qu'il consiste à dissoudre l'hydantoïne chirale (I) à dédoubler dans une solution alcaline puis à ajouter l'énantiomère de l'α-méthylbenzylamine à raison d'au moins un équivalent molaire pour une mole d'hydantoïne à dédoubler.

3. Procédé selon la revendication 2, caractérisé en ce que la solution alcaline est une solution aqueuse ammoniacale contenant au moins trois équivalents d'ammoniaque par mole d'hydantoïne à dédoubler.

4. Procédé selon la revendication 2, caractérisé en ce que la solution alcaline est une solution aqueuse contenant de 0,3 à 0,75 équivalents d'hydroxyde alcalin par mole d'hydantoïne à dédoubler.

5. Procédé selon la revendication 4, caractérisé en ce que la solution aqueuse d'hydroxyde alcalin est additionnée d'un co-solvant choisi dans le groupe formé par les alcools hydrosolubles et l'acétone.

6. Procédé selon la revendication 5, caractérisé en ce que le co-solvant est l'acétone, mise en oeuvre à raison de 0,2 à 10 volumes pour un volume de solution aqueuse.

7. Procédé selon l'une quelconque des revendications 4 à 6, caractérisé en ce que l'hydroxyde alcalin mis en oeuvre est l'hydroxyde de sodium.

8. Procédé selon la revendication 3, caractérisé en ce que l'hydantoïne à dédoubler est la (+/-)5-phényl-5-éthyl hydantoïne.

9. Procédé selon la revendication 4, caractérisé en ce que l'hydantoïne à dédoubler est la (+/-)5-phényl-5-éthyl-hydantoine.

10. Procédé selon la revendication 4, caractérisé en ce que l'hydantoïne à dédoubler est la (+/-)5-phényl-5-méthyl-hydantoïne.

11. Procédé selon la revendication 4, caractérisé en ce que l'hydantoine à dédoubler est la (+/-)5-p-tolyl-5-méthyl-hydantoine.

12. Procédé selon la revendication 4, caractérisé en ce que l'hydantoïne à dédoubler est la (+/-)5-phényl-5-n-propyl-hydantoïne.

13. Procédé selon la revendication 4, caractérisé en ce que l'hydantoïne à dédoubler est la (+/-)5-phényl-5-i-propyl-hydantoïne.

14. Procédé selon la revendication 6, caractérisé en ce que l'hydantoïne à dédoubler est la (+/-)-5-phénéthyl-5-méthyl-hydantoïne.

15. Procédé selon la revendication 5 et la revendication 6, caractérisé en ce que l'hydantoïne à dédoubler est la (+/-)-5-phényl-5-éthyl-hydantoine.

16. Procédé selon la revendication 4, caractérisé en ce que l'hydantoïne à dédoubler est la (+/-)5-(2-thiényl)-5-éthyl-hydantoïne.

17. Procédé selon la revendication 5 ou la revendication 6, caractérisé en ce que l'hydantoïne à dédoubler est la (+/-)5-(3,4-dichlorobenzyl)-5-méthyl-hydantoïne.

## Patentansprüche

1. Verfahren zur Racemattrennung von chiralen Hydantoinen, wobei das zu trennende Hydantoin in einem Lösungsmittel gelöst wird, der Lösung ein Enantiomer von α-Methylbenzylamin in definiertem Verhältnis zugegeben wird und anschließend das erhaltene diastereomere Salz isoliert und mit einer sauren Lösung behandelt wird, um das Hydantoin-Enantiomer auszukristallisieren, dadurch gekennzeichnet, daß das Hydantoin die allgemeine Formel (I) 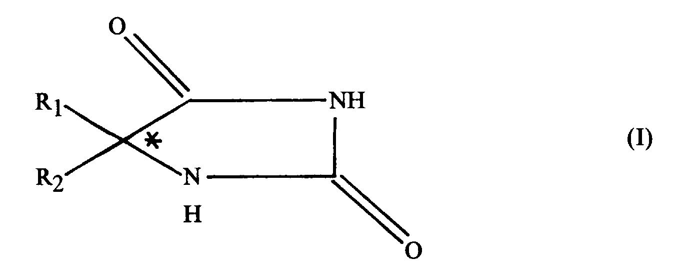 aufweist, in der:
R₁ ein kurzkettiges Alkylradikal mit 1 bis 5 Kohlenstoffatomen in gerader oder verzweigter Kette ist,
R₂ ein mit kurzkettigen Alkyl- oder Alkoxyradikalen, die 1 bis 5 Kohlenstoff- oder gleiche oder verschiedene Halogenatome aufweisen, eventuell mono-, di- oder trisubstituiertes Phenylradikal oder ein Heteroalkylradikal mit einem 5- bis 7-gliedrigem Ring, in dem das einzige Heteroatom Stickstoff Sauerstoff oder Schwefel ist, oder aber ein Aralkylradikal ist, dessen Alkylteil ein oder zwei Kohlenstoffatome umfallt und dessen Arylring eine mit kurzkettigen Akyl- oder Alkoxyradikalen oder gleichen oder verschiedenen Halogenatomen eventuell mono-, di- oder trisubstituierte Phenylgruppe ist und wobei das Lösungsmittel eine alkalische Lösung ist.

2. Verfahren gemäß Anspruch 1, dadurch gekennzeichnet, daß das zu trennende chirale Hydantoin (I) in einer alkalischen Lösung gelöst wird und anschließend das Enantiomer von α-Methylbenzylamin in einem Verhältnis von mindestens einem Moläquivalent pro Mol zu trennendem Hydantoin zugegeben wird.

3. Verfahren gemäß Anspruch 2, dadurch gekennzeichnet, daß die alkalische Lösung eine wässrige Ammoniaklösung ist, die mindestens drei Äquivalente Ammoniak pro Mol zu trennendem Hydantoin enthält.

4. Verfahren gemäß Anspruch 2, dadurch gekennzeichnet, daß die alkalische Lösung eine wässrige Lösung ist, die 0,3 bis 0,75 Äquivalente Alkalihydroxid pro Mol zu trennendem Hydantoin enthält.

5. Verfahren gemäß Anspruch 4, dadurch gekennzeichnet, daß zu der wässrigen Alkalihydroxidlösung ein zweites Lösungsmittel zugegeben wird, das aus der Gruppe, bestehend aus wasserlöslichen Alkoholen und Aceton ausgewählt wird.

6. Verfahren gemäß Anspruch 5, dadurch gekennzeichnet, daß das zweite Lösungsmittel Aceton, verwendet im Verhältnis von 0,2 bis 10 Volumenteilen pro Volumenteil wässriger Lösung, ist.

7. Verfahren gemäß einem der Ansprüche 4 bis 6, dadurch gekennzeichnet, daß das eingesetzte Alkalihydroxid Natriumhydroxid ist.

8. Verfahren gemäß Anspruch 3, dadurch gekennzeichnet, daß das zu trennende Hydantoin (+/-)5-Phenyl-5-ethylhydantoin ist.

9. Verfahren gemäß Anspruch 4, dadurch gekennzeichnet, daß das zu trennende Hydantoin (+/-)5-Phenyl-5-ethylhydantoin ist.

10. Verfahren gemäß Anspruch 4, dadurch gekennzeichnet, daß das zu trennende Hydantoin (+/-)5-Phenyl-5-methylhydantoin ist.

11. Verfahren gemäß Anspruch 4, dadurch gekennzeichnet, daß das zu trennende Hydantoin (+/-)5-p-Tolyl-5-methylhydantoin ist.

12. Verfahren gemäß Anspruch 4, dadurch gekennzeichnet, daß das zu trennende Hydantoin (+/-)5-Phenyl-5-n-propylhydantoin ist.

13. Verfahren gemäß Anspruch 4, dadurch gekennzeichnet, daß das zu trennende Hydantoin (+/-)5-Phenyl-5-i-propylhydantoin ist.

14. Verfahren gemäß Anspruch 6, dadurch gekennzeichnet, daß das zu trennende Hydantoin (+/-)5-Phenethyl-5-methylhydantoin ist.

15. Verfahren gemäß Anspruch 5 und Anspruch 6, dadurch gekennzeichnet, daß das zu trennende Hydantoin (+/-)5-Phenyl-5-ethylhydantoin ist.

16. Verfahren gemäß Anspruch 4, dadurch gekennzeichnet, daß das zu trennende Hydantoin (+/-)5-(2-Thienyl)-5-ethylhydantoin ist.

17. Verfahren gemäß Anspruch 5 oder 6, dadurch gekennzeichnet, daß das zu trennende Hydantoin (+/-)5-(3,4-Dichlorbenzyl)-5-methylhydantoin ist.

## Claims

1. A method of resolution of chiral hydantoins comprising dissolving the hydantoin to be resolved in a solvent, adding to the resultant solution an enantiomer of α-methylbenzylamine in defined proportion and then isolating the diastereoisomeric salt obtained and treating it with an acid solution in order to crystallize the enantiomer of the hydantoin, characterized in that the hydantoin has the general formula I 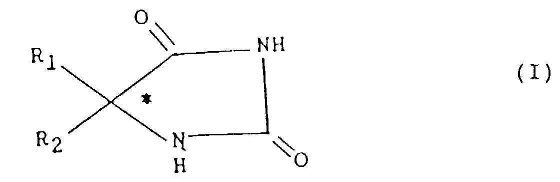 in which:
R₁ designates a lower alkyl radical comprising 1 to 5 carbon atoms in linear or branched chain,
R₂ designates a phenyl radical, possibly mono, di or tri-substituted by lower alkyl or alkoxy radicals or halogen atoms, whether identical or different, or a heteroaryl radical containing a ring of 5 to 7 members in which the only heteroatom is nitrogen, oxygen or sulfur, or else an aralkyl radical the alkyl part of which comprises one or two carbon atoms and the aryl cycle is a phenyl group, possibly mono-, di or tri-substituted by radicals which are lower alkyl or alkoxy radicals or halogen atoms, whether identical or different and the solvent is an alkaline solution.

2. A method according to claim 1, characterized by the fact that it comprises dissolving the chiral hydantoin (I) to be resolved in an alkaline solution and then adding the enantiomer of α-methylbenzylamine in the amount of at least one molar equivalent to one mol of hydantoin to be resolved.

3. A method according to claim 2, characterized by the fact that the alkaline solution is an ammoniacal aqueous solution containing at least three ammonia equivalents per mol of hydantoin to be resolved.

4. A method according to claim 2, characterized by the fact that the alkaline solution is an aqueous solution containing 0.3 to 0.75 equivalents of alkaline hydroxide per mol of hydantoin to be resolved.

5. A method according to claim 4, characterized by the fact that to the aqueous alkaline hydroxide solution there is added a co-solvent selected from the group consisting of water-soluble alcohols and acetone.

6. A method according to claim 5, characterized by the fact that the co-solvent is acetone, used in an amount of 0.2 to 10 parts by volume to one part by volume of aqueous solution.

7. A method according to any of claims 4 to 6, characterized by the fact that the alkaline hydroxide used is sodium hydroxide.

8. A method according to claim 3, characterized in that the hydantoin to be resolved is (+/-)-5-phenyl-5-ethyl-hydantoin.

9. A method according to claim 4, characterized in that the hydantoin to be resolved is (+/-)-5-phenyl-5-ethyl-hydantoin.

10. A method according to claim 4, characterized in that the hydantoin to be resolved is (+/-)-5-phenyl-5-methyl hydantoin.

11. A method according to claim 4, characterized in that the hydantoin to be resolved is (+/-)-5-p-tolyl-5-methyl hydantoin.

12. A method according to claim 4, characterized in that the hydantoin to be resolved is (+/-)-5-phenyl-5-n-propyl hydantoin.

13. A method according to claim 4, characterized in that the hydantoin to be resolved is (+/-)-5-phenyl-5-i-propyl hydantoin.

14. A method according to claim 4, characterized in that the hydantoin to be resolved is (+/-)-5-phenethyl-5-methyl hydantoin.

15. A method according to claim 5 and claim 6, characterized in that the hydantoin to be resolved is (+/-)-5-phenyl-5-ethyl hydantoin.

16. A method according to claim 4, characterized in that the hydantoin to be resolved is (+/-)-5-(2-thienyl)-5-ethyl hydantoin.

17. A method according to claim 5 or 6, characterized in that the hydantoin to be resolved is (+/-)-5-(3,4-dichlorobenzyl)-5-methyl-hydantoin.
